# EUROPÄISCHE PATENTSCHRIFT

(11) **EP 1 460 947 B1**
(45) Veröffentlichungstag und Bekanntmachung des Hinweises auf die Patenterteilung: **29.09.2010**
(21) Anmeldenummer: 02796513.6
(22) Anmeldetag: 18.12.2002
(51) Int. Cl.: A61B 17/22

(54) **PRÜFUNG UND ÜBERWACHUNG EINER STOSS-BZW. DRUCKWELLENQUELLE**
TESTING AND MONITORING OF A SHOCK WAVE OR PRESSURE WAVE SOURCE
CONTROLE ET SURVEILLANCE D'UNE SOURCE D'ONDES DE CHOC OU DE PRESSION

(30) Priorität: 19.12.2001 DE 10162595
(43) Veröffentlichungstag der Anmeldung: 29.09.2004
(73) Patentinhaber: Dornier MedTech Systems GmbH, 82234 Wessling (DE)
(72) Erfinder: EIZENHÖFER, Harald, 82229 Seefeld (DE)
(74) Vertreter: Grünecker, Kinkeldey, Stockmair & Schwanhäusser Anwaltssozietät
(86) Internationale Anmeldenummer: PCT/DE2002/004638
(87) Internationale Veröffentlichungsnummer: WO 2003/052373

(56) Entgegenhaltungen:
- EP-A- 0 256 202
- DE-A- 19 640 050

## Beschreibung

Die Erfindung bezieht sich auf die Prüfung und Überwachung der Funktion einer Stoss- bzw. Druckwellenquelle, wie sie beispielsweise im Rahmen der elektromagnetischen Stosswellen-Lithotripsie zur Anwendung kommt.

Zur Qualitätssicherung und Funktionskontrolle von in der Medizin verwendeten Stoss- bzw. Druckwellenquellen - im folgenden kurz nur Stosswellenquellen genannt - werden von Endtest- und Service - Technikern Druckmessungen mit Hilfe von piezoelektrischen Sensoren und auch Zertrümmerungstests mit synthetischen Steinen durchgeführt. DE-A-196 40 505 zeigt eine Vorrichtung für solche Messungen.

Beide Testverfahren sind mit einer Reihe von Nachteilen behaftet, die hier nur beispielsweise erwähnt und kommentiert werden sollen. Beide Testverfahren benötigen ein mit Wasser gefülltes Testgefäss, wobei der Gasgehalt im Wasser einer ständigen strengen Kontrolle zu unterziehen ist. Desweiteren setzt die piezoelektrische Messtechnik eine umfassende Erfahrung im Umgang mit Oszilloskopen voraus. Im übrigen ist die Qualität der in den Zertrümmerungstests verwendeten Gipskugeln nicht gleichbleibend auf dem zu erwartetenden Niveau; sogenannte Einweichzeiten müssen abgewartet und dabei streng eingehalten werden.

Der Erfindung liegt daher die Aufgabe zugrunde, zur Prüfung bzw. Überwachung einer eingangs genannten Stosswellenquelle unter Vermeidung der aufgezeigten Nachteile eine wesentlich einfachere, robuste und zuverlässige Messeinrichtung vorzuschlagen, welche ausserdem leicht zu bedienen ist und auch hinsichtlich der Wartung kaum Probleme aufwirft. Des weiteren soll für den eingangs genannten Zweck eine Überwachungsvorrichtung vorgeschlagen werden, die permanent an den betreffenden Geräten verbleiben und zur ständigen Überwachung der Geräte, auch während ihrer Einsätze dienen kann.

Gelöst ist diese Aufgabe im wesentlichen dadurch, dass die erfindungsgemässe Prüf- und Überwachungseinrichtung unter anderem aus einem passiven, nichtlinearen Stosswellenimpulse von nur einigen Mikro-Sekunden Pulsdauer in einen erheblich niedrigeren Frequenzbereich von ca. 0,1 bis 1 kHz transformiert, dessen Schwingungen dann sensiert und ausgewertet werden.

Das passive, nichtlineare Übertragungsglied mit der Eigenschaft der spektralen Konversion kann praktisch jedes schwingfähige Gebilde sein. Anschaulich kann von einer Glocke oder von einer Stimmgabel gesprochen werden, die, von einem Impuls angeregt, Eigenschwingungen vollführen.

Der analytische Teil der im Rahmen der Erfindung zu verwendenden Sensorik besteht darin, aus dem registrierten Schwingverhalten des Übertragungsgliedes die richtigen Rückschlüsse auf die Grösse des Anregungsimpulses zu ziehen. Hierzu können die "Lautstärke", die spektrale Zusammensetzung der Schwingungen, das Abklingverhalten einzelner Spektralanteile etc. einzeln oder in Kombination ausgewertet werden.

Der am Übertragungsglied anzubringende Sensor kann bevorzugt ein preisgünstiger Airbag-Auslöse-Beschleunigungssensor sein, wenn die Beschleunigungen von Strukturen des Gebildes gemessen werden sollen. Zur Messung des Schalldruckes würde ein entsprechendes Mikrophon genügen. Vorteilhafter sind jedoch in dem vorliegenden Zusammenhang die vorerwähnten Airbag-Sensoren, bei denen im Gegensatz zu Mikrophonen die Signalaufbereitung, die Signalfilterung und teilweise sogar schon eine digitale Stromschnittstelle auf einem Chip integriert sind. Daher ist bei Airbag-Sensoren ein geringer Gesamtaufwand an nachgeschalteter Elektronik bis hin zur Darstellung der Messgrösse zu erwarten. Für den hochintegrierten Sensor-Chip mit integriertem Hochpassfilter spricht nicht zuletzt auch die zu erwartende grosse Unempfindlichkeit gegen elektromagnetische Störungen, die bei mit elektrischen Impulsen im Megawatt-Bereich betriebenen Stosswellenquellen stets präsent sind.

In einer weiteren erfindungsgemässen Ausführungsform kann als passives, nichtlineares Übertragungsglied ein Medium mit hoher Absorption des einlaufenden akustischen Impulses dienen. In diesem Falle ist das anschauliche Beispiel ein Sandsack, der bei Beschuss durch ein Projektil dieses vollständig abbremst. Wegen der Impulserhaltung ist über die Beschleunigung des Absorbers im Rückschluss der Impuls der eingestrahlten akustischen Stosswelle messbar.

Der Sensor ist auch hier direkt am Absorber anzubringen. Um hohe Beschleunigungswerte, dh. ein gutes Signal/Rauschverhältnis zu erzielen, muss die Gesamtmasse Absorber/Sensor klein gehalten werden. Diese Einschränkung ist bei einem resonanzfähigen Gebilde nicht erforderlich, da dort die Masse des Sensor-Chips das Schwingverhalten des Gebildes nur gering beeinflusst oder sogar beim Design des resonanzfähigen Gebildes berücksichtigt werden kann.

Besondere Ausgestaltungen im Rahmen der Erfindung sind der nachfolgenden Abbildungsbeschreibung sowie den Patentansprüchen zu entnehmen.

Der wesentliche Vorteil der Erfindung besteht darin, dass niederfrequente Schwingungen mit kommerziellen Airbag-Auslöse-Sensoren erheblich leichter registrierbar sind als die hochfrequenten direkten Stosswellenimpulse.

### Abbildungsbeschreibung

In den Abbildungen ist die Erfindung anhand von Ausführungsbeispielen zeichnerisch erläutert.

Es zeigen :
- Abb. 1: eine Stosswellenquelle mit einem Koppelbalg, der mit einem geeigneten Fluid, vorteilhaft mit Wasser gefüllt ist, und an dessen Stirn- seite ein resonanzfähiges, stimmgabelähnliches Gebilde mit seinem unteren Schenkel am Koppelbalg anliegt.
- Abb. 2: einen Koppelbalg, dessen Stirnseite mit einem zylinderförmigen Absorber axialsymmetrisch in Berührung steht.
- Abb. 3: als schwingungs- bzw. resonanzfähiges Gebilde einen Edelstahldraht, der im Rahmen der erfindungsgemässen Messeinrichtung mit einem Teil seiner Länge in den Stosswellen- Pfad hineinragt.

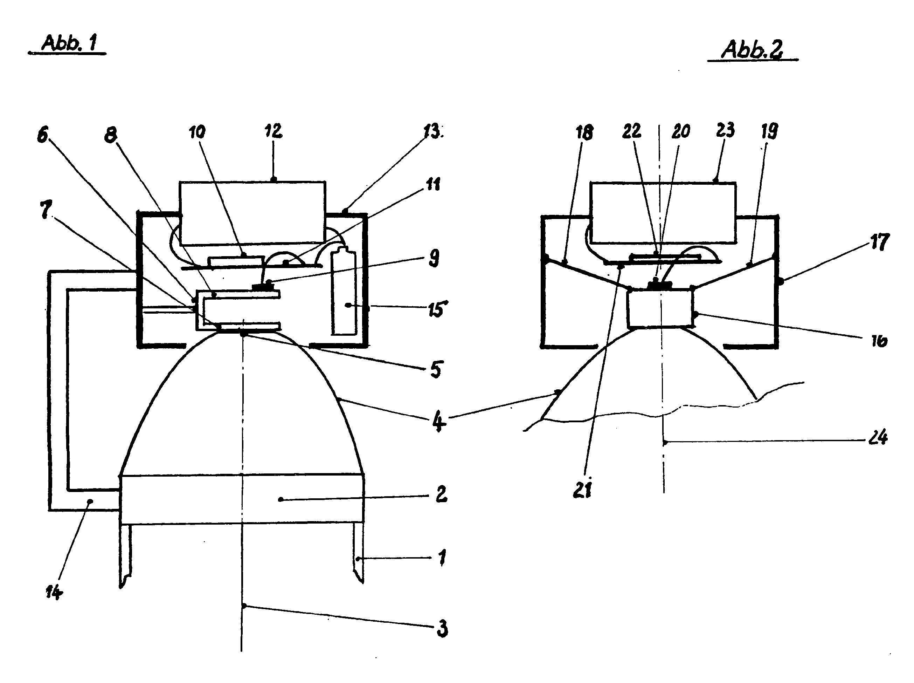

Die Abbildung 1 zeigt einen Therapiekopf 1- beispielsweise zur Durchführung der extrakorporalen Lithotripsie - der unter anderem eine Stosswellenquelle 2 enthält. Diese ist über die Leitung 3 an eine nicht gesondert dargestellte elektrische Versorgungseinheit abgeschlossen. Patientenseitig ist die Stosswellenquelle 2 mit einem wassergefüllten Koppelbalg 4 abgeschlossen, der während der Therapie mit der betreffenden Körperstelle des Patienten in Berührung steht, das heisst, über den normalerweise ohne die Messeinrichtungen gemäss den Abbildungen 1 und 2 die Stosswellen in den Körper des Patienten eingeleitet werden.

Zur erfindungsgemässen Vermessung, Überwachung und/oder Kontrolle der Stosswellenquelle 2 steht die Stirnseite 5 des Koppelbalgs 4 mit einem resonanzfähigen Gebilde in Form einer liegenden Stimmgabel 6 in enger Berührung, derart, dass der untere Schenkel 7 der Stimmgabel 6 den Koppelbalg 4 stirnflächig berührt, während der obere, freie Schenkel 8 - vorzugsweise an seinem freien Ende- einen fest angebrachten Beschleunigungssensor 9 aufweist. Dieser ist mit einer diverse Chips 10 tragenden Elektronikplatine 11 verbunden. Ein mit der Platine 11 in Verbindung stehendes Display 12 zeigt dem Betrachter die Messergebnisse auf der Basis der vom Beschleunigungssensor 9 registrierten Signale.

Ein Schutzgehäuse 13 ist mittels einer Halterung 14 fest, aber lösbar mit dem Therapiekopf 1 verbunden. Innerhalb des Schutzgehäuses 13 kann noch eine Batterie 15 untergebracht sein, die die Elektronik der Platine 11, den Beschleunigungssensor 9 und das Display 12 mit Spannung versorgt.

Die Abbildung 2 zeigt das gleiche Testobjekt wie Abbildung 1 hinsichtlich der Bezugszeichen 1 bis 4. Auch die Messeinrichtung ist praktisch identisch bezüglich der Bezugszeichen 9 bis 15.

Im Unterschied zu Abbildung 1 ist bei der Messeinrichtung gemäss Abbildung 2 das resonanzfähige Gebilde ein Absorber 16. Dieser ist innerhalb eines Schutzgehäuses 17 an Fäden bzw. an Federn 18 und 19 aufgehängt.

Am Absorbers 16 ist ein Beschleunigungssensor 20 angebracht, der mit einer Elektronikplatine 21 verbunden ist. Die elektronische Messeinrichtung ist im übrigen identisch mit derjenigen der Abbildung 1. Das Display trägt hier das Bezugszeichen 23. Auf der Platine 21 befinden sich Chips 22. Die Längsachse des Therapiekopfes trägt das Bezugszeichen 24.

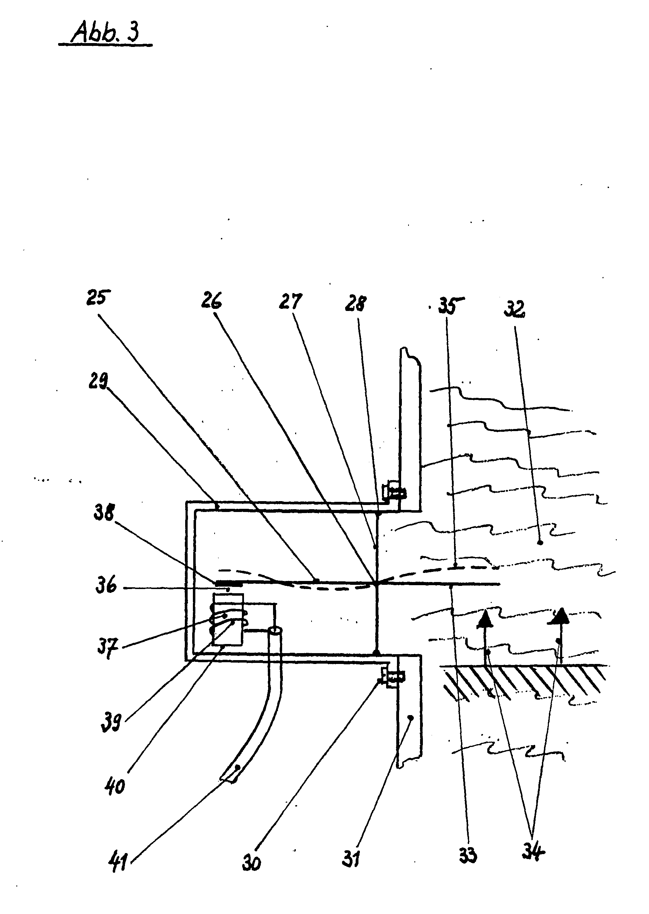

Die Abbildung 3 zeigt eine Messeinrichtung im Rahmen der Erfindung, die sich im wesentlichen von den Lösungsvorschlägen der Abbildungen 1 und 2 dadurch unterscheidet, dass das resonanzfähige Gebilde nicht ausschliesslich von aussen am Therapiekopf anliegt, sondern teilweise in diesen hineinragt, und zwar vorzugsweise in das Fluid zwischen der elektromagnetischen Stosswellenquelle und der akustischen Linse.

Das erfindungsgemässe resonanzfähige Gebilde gemäss Abbildung 3 ist ein Edelstahldraht 25, der möglichst gehärtet ist und der in seiner Durchführung 26 durch eine Edelstahlmembrane 27 möglichst hochwertig mit dieser verschweisst ist. Die Membrane 27 ist ihrerseits in dem umlaufenden Bereich 28 ebenfalls möglichst hochwertig mit einem Gehäuse 29 dicht verschweisst. Das Gehäuse 29 ist mittels Schrauben 30 dicht mit dem Gehäuse 31 eines Therapiekopfes 32 verbunden.

Wenn der in den Therapiekopf 32 hineinragende Teil 33 des Drahtes 25 durch eine Stosswelle 34 einen Impuls erfährt, beginnt der Draht 25 zu schwingen. Die gestrichelte Linie 35 soll diese Schwingung in übertriebener Darstellung verdeutlichen.

Wenn die Schwingungen des Drahtes 25 mit einer sogenannten Pick up-Spule 37 erfasst werden, wie in der Abbildung 3 dargestellt, dann muss der Draht 25 im Messbereich 36 eine Permeabilität besitzen, die wesentlich grösser als 1 ist. Dies ist durch ein entsprechend ferritisches Metallteil 38 gewährleistet, welches am Draht 25 im Messbereich 36 zu befestigen ist. Der Signalaufnehmer 37 besteht aus einer Spule 39 sowie aus einem Permanentmagneten 40. Die registrierten Messwerte können über eine Leitung 41 einer elektronischen Verarbeitung zur Darstellung der Messergebnisse zugeführt werden.

Weitere Abtastmethoden, insbesondere optischer Art sind vorstellbar.

## Patentansprüche

1. Einrichtung zur Prüfung und/oder Überwachung von Stoss- bzw. Druckwellenquellen, wie sie beispielsweise im Rahmen der elektromagnetischen Stosswellen-Lithotripsie zur Anwendung kommen, **gekennzeichnet durch** ein passives, nichtlineares schwingungs- bzw. resonanzfähiges Gebilde (6,16,25), welches **durch** eine Stoss- bzw. Druckwelle anregbar ist, wobei die sehr kurzzeitigen Stoss- bzw. Druckwellenimpulse von einigen Mikro-Sekunden Pulsdauer in einen wesentlich niedrigeren Frequenzbereich von vorzugsweise 0,1 bis 1 kHz transformiert werden und eine elektronische Einrichtung (9,10,11;20,21,22;37,39,40) vorgesehen ist, welche die Schwingungen des Gebildes (6, 16, 25) registrieren und analysieren kann, und die Messergebnisse auf einem Display (12, 23) abgelesen werden können.

2. Einrichtung nach Anspruch 1, **dadurch gekennzeichnet, dass** das schwingungs- bzw. resonanzfähige Gebilde (6) die Grundform einer Stimmgabel hat, von der ein Schenkel (7) stirnseitig mit dem mit einem geeigneten Fluid, vorteilhaft mit Wasser gefüllten Koppelbalg (4) einer Stoss- bzw. Druckwellenquelle in enger, flächiger Berührung steht, und dessen anderer Schenkel (8) an seinem freien Ende einen Beschleunigungssensor (9) aufweist, der die von ihm aufgenommenen Schwingungssignale zur Verarbeitung an Chips (10) weiterleitet, die auf einer elektronischen Platine (11) angeordnet sind, wobei letztere zur Sichtbarmachung der Messergebnisse mit einem Display (12) verbunden sein kann.

3. Einrichtung nach den Ansprüchen 1 und 2, **dadurch gekennzeichnet, dass** das schwingungs- bzw. resonanzfähige Gebilde ein Absorber (16) ist, der mit seiner einen Stirnseite den Koppelbalg (4) eng berührt, und an dem ein Beschleunigungssensor (20) befestigt ist, der die registrierten Signale an die Elektronik (21,22) zur Analyse bzw. zur Verarbeitung weiterleitet, während der Absorber (16) in einem die Überwachungseinrichtung überdeckenden Schutzgehäuse (17) an Fäden oder an Federn (18,19) gleichsam als seismische Masse aufgehängt ist.

4. Einrichtung nach Anspruch 1, **dadurch gekennzeichnet, dass** das schwingungs- bzw. resonanzfähige Gebilde ein gehärteter Edelstahldraht (25) ist, der in seiner Durchführung (26) durch eine Edelstahlmembrane (27) mit dieser verschweisst ist, während die Membrane (27) ihrerseits in dem umlaufenden Bereich (28) mit einem Gehäuse (29) ebenfalls verschweisst ist, welches seinerseits über Schrauben (30) dicht mit dem Gehäuse (31) eines Therapiekopfes (32) verbunden ist, wobei der nach innen gerichtete Teil (33) des Edelstahldrahtes (25) in das flüssige Koppelmedium eines Therapiekopfes (32) ragt, derart, dass der Draht (25) durch Stosswellen (34) zu Schwingungen anregbar ist, wobei im Messbereich (36) mittels eines Signalaufnehmers (37) in Form einer Spule (39) und eines Permanentmagneten (40) eine Signalaufnahme erfolgen kann, die über eine Leitung (41) einer elektronischen Verarbeitung zur Darstellung der Messergebnisse zugeführt werden können.

5. Einrichtung nach Anspruch 4, **dadurch gekennzeichnet, dass** die Signalaufnahme nach dem Prinzip der elektromagnetischen Induktion, d.h. gemäss der zeitlichen Änderung der magnetischen Durchflutung einer Spule erfolgt.

6. Einrichtung nach einem oder mehreren der Ansprüche 1 bis 4, **dadurch gekennzeichnet, dass** die Signalaufnahme optisch erfolgt.

## Claims

1. Device for testing and/or monitoring shock or pressure wave sources, such as used in the field of electromagnetic shock wave lithotripsy, **characterised by** a passive, non-linear construction (6, 16, 25) capable of vibrating or resonating, which can be excited by a shock or pressure wave, and the very brief shock or pressure wave pulses of a pulse duration of a few micro-seconds are transformed into a significantly lower frequency range of preferably 0.1 to 1 kHz, and an electronic device (9, 10, 11; 20, 21, 22; 37, 39, 40) is provided which is able to record and analyse the vibrations of the construction (6, 16, 25), and a reading of the measurement results can be taken from a display (12, 23).

2. Device as claimed in claim 1, **characterised in that** the vibrating or resonating construction (6) has the basic shape of a tuning fork, one leg (7) of which sits with its end face in close planar contact with the coupling bellows (4) of a shock or pressure wave source filled with an appropriate fluid, advantageously water, and the other leg (8) of which has an acceleration sensor (9) at its free end which forwards the vibration signals recorded by it to chips (10) disposed on an electronic circuit board (11) for processing, and the latter can be connected to a display (12) to enable the measurement results to be visualised.

3. Device as claimed in claims 1 and 2, **characterised in that** the vibrating or resonating construction is an absorber (16) which sits in close contact with the coupling bellows (4) by means of its one end face and to which an acceleration sensor (20) is attached which forwards the recorded signals to the electronic system (21, 22) for analysis and processing, whilst the absorber (16) is suspended on threads or on springs (18, 19) serving as a seismic mass, so to speak, in a protective housing (17) covering the monitoring device.

4. Device as claimed in claim 1, **characterised in that** the vibrating or resonating construction is a hardened stainless steel wire (25), which is welded in its passage (26) through a stainless steel membrane (27) to the latter, whilst the membrane (27) is in turn likewise welded to a housing (29) in the surrounding region (28), which is in turn tightly connected to the housing (31) of a therapy head (32) by means of screws (30), and the inwardly directed part (33) of the stainless steel wire (25) extends into the liquid coupling medium of a therapy head (32) so that the wire (25) can be excited by shock waves (34) to induce vibrations, and a signal can be recorded in the measurement region (36) by means of a signal recorder (37) in the form of a coil (39) and a permanent magnet (40), which can be forwarded via a line (41) to an electronic processing system to enable the measurement results to be displayed.

5. Device as claimed in claim 4, **characterised in that** the signal is recorded using the principle of electromagnetic induction, i.e. based on the change in the magnetic flux through a coil as a function of time.

6. Device as claimed in one or more of claims 1 to 4, **characterised in that** the signal is optically recorded.

## Revendications

1. Dispositif pour contrôler et/ou surveiller des sources d'ondes de choc ou de pression telles qu'elles sont utilisées par exemple dans le cadre de la lithotripsie électromagnétique par ondes de choc, **caractérisé par** une structure passive non linéaire vibrante et résonante (6, 16, 25) qui est apte à être excitée par une onde de choc ou de pression, étant précisé que les impulsions d'ondes de choc ou de pression très courtes d'une durée de quelques microsecondes sont transformées en une plage de fréquence nettement plus faible et de préférence de 0,1 à 1 kHz, et il est prévu un dispositif électronique (9, 10, 11 ; 20, 21, 22 ; 37, 39, 40) qui peut enregistrer et analyser les vibrations de la structure (6, 16, 25), et les résultats de mesure peuvent être lus sur un affichage (12, 23).

2. Dispositif selon la revendication 1, **caractérisé en ce que** la structure vibrante ou résonante (6) a la forme de base d'un diapason dont une branche (7) est en contact étroit et plan, côté frontal, avec le coussin de couplage (4), rempli d'un fluide approprié et avantageusement d'eau, d'une source d'ondes de choc ou de pression, tandis que son autre branche (8) présente à son extrémité libre un capteur d'accélération (9) qui transmet les signaux de vibration qu'il reçoit à des puces (10) disposées sur une carte électronique (11), en vue de leur traitement, lesdites puces (10) pouvant être reliées à un affichage (12) afin de visualiser les résultats de mesure.

3. Dispositif selon les revendications 1 et 2, **caractérisé en ce que** la structure vibrante ou résonante est constituée par un absorbeur (16) qui touche étroitement le soufflet de couplage (4), avec un côté frontal, et auquel est fixé un capteur d'accélération (20) qui transmet les signaux enregistrés au système électronique (21, 22) en vue de leur analyse ou traitement, tandis que l'absorbeur (16) est suspendu en quelque sorte comme masse sismique à des fils ou à des ressorts (18, 19) dans un boîtier de protection (17) qui couvre le dispositif de surveillance.

4. Dispositif selon la revendication 1, **caractérisé en ce que** la structure vibrante ou résonante est constituée par un fil en acier spécial trempé (25) qui, dans son passage (26) à travers une membrane en acier spécial (27), est soudé à celle-ci tandis que la membrane (27) est elle-même soudée dans la zone circonférentielle (28) à un boîtier (29) qui est quant à lui relié de manière étanche au boîtier (31) d'une tête thérapeutique (32) par l'intermédiaire de vis (30), étant précisé que la partie (33) du fil en acier trempé (25) dirigée vers l'intérieur entre dans l'agent de couplage liquide d'une tête de traitement (32), de sorte que des ondes de choc (34) peuvent faire vibrer le fil (25), et que dans la zone de mesure (36) un enregistrement de signaux peut avoir lieu à l'aide d'un capteur de signaux (37) en forme de bobine (39) et d'un aimant permanent (40), lequel enregistrement de signaux peut être transmis par l'intermédiaire d'une ligne (41) en vue d'un traitement électronique, pour visualiser les résultats de mesure.

5. Dispositif selon la revendication 4, **caractérisé en ce que** l'enregistrement de signaux se fait suivant le principe de l'induction électromagnétique, c'est-à-dire suivant la variation dans le temps du flux magnétique qui traverse une bobine.

6. Dispositif selon l'une au moins des revendications 1 à 4, **caractérisé en ce que** l'enregistrement de signaux se fait par voie optique.
